# EUROPEAN PATENT APPLICATION

(11) **EP 3 766 464 A1**
(43) Date of publication of application: **20.01.2021**
(21) Application number: 19767277.7
(22) Date of filing: 15.03.2019
(51) Int. Cl.: A61F 13/02, A61F 13/00

(54) **WOUND-COVERING MATERIAL**

(30) Priority: 16.03.2018 JP 2018050041
(71) Applicant: Nichiban Co., Ltd., Bunkyo-ku Tokyo 112-8663 (JP)
(72) Inventor: IKAI, Tomonori, Tokyo 112-8663 (JP); NAKANO, Shigekazu, fuji-shi, Shizuoka 419-0204 (JP)
(74) Representative: SSM Sandmair
(86) International application number: PCT/JP2019/010906
(87) International publication number: WO 2019/177156

(57) **Abstract**

To provide a wound-covering material that improves visibility of a wound site and has a high exudate absorbing rate and an appropriate exudate holding amount.

A wound-covering material includes a super absorbent pad having a laminated structure of polyester nonwoven fabric layer/water-absorbing layer. The water-absorbing layer includes a super absorbent fiber, a polypropylene fiber, and a polyethylene binder. The super absorbent pad has an opening passing therethrough.

## Description

### Technical Field

The present invention relates to a wound-covering material used for covering a wound site on a skin to prepare a healing environment.

### Background Art

Wound-covering materials are roughly classified into a dressing material (modern wound-covering material) and a medical material such as a gauze (classical wound-covering material). The former generally refers to a medical material used for covering and protecting a wound caused by trauma, surgery, or the like, a burn, a bedsore, or the like during treatment thereof, and maintaining a moist environment of the wound to prepare an environment suitable for wound healing (hereinafter, a wound-covering material here refers to this type of medical material).

In general, a wound-covering material includes a transparent film, a pressure-sensitive adhesive, a pad such as an opaque nonwoven fabric, a urethane foam, and the like. For example, Patent Literature 1 discloses a wound bandage formed by laminating a moisture permeable polyurethane layer and a nonwoven fabric layer containing a hydrophobic fiber and a super absorbent fiber (SAF).

Materials and compositions of wound-covering materials proposed so far have been variously selected from a viewpoint of not only protecting a wound site but also mainly absorbing and holding exudate derived from a wound and maintaining a wound surface in a moist environment to prepare a wound healing environment. In addition, it is required to select an appropriate type of wound-covering material, to change a wound-covering material, or the like according to a healing process. However, many wound-covering materials that have been proposed/marketed so far have poor transparency, and it is often difficult to observe (visually) a progress of a wound surface in a state where the wound materials are stuck.

Patent Literature 2 discloses a wound-covering material including a lattice containing a material having at least one slit. The wound-covering material of Patent Literature 2 is intended to visualize a wound through an opening formed by opening the slit by applying a force perpendicular to a longitudinal axis of the slit.

### Citation List

### Patent Literature

Patent Literature 1: JP 2000-175959 A
Patent Literature 2: JP 2010-504835 A

### Summary of Invention

### Technical Problem

Many conventional wound-covering materials that have been proposed/marketed so far make it difficult to observe/confirm (visually) a healing condition of a wound surface and signs of infectious diseases and the like, and make it difficult to find a guideline for changing the covering materials. In a mode in which a slit is disposed as disclosed in the cited document 2, it is necessary to extend a wound-covering material such that the slit serves as an opening, and a wound surface cannot be confirmed at a glance. In a case where it is assumed that a hole that is open all the time is formed in a wound-covering material such that a wound site can be confirmed all the time, it is difficult to protect a wound located in the hole, to hold exudate, and further to maintain a moist environment suitable for wound healing, and the strength of the covering material itself or shape retention thereof may be affected.

Even with a conventional wound-covering material, when the amount of exudate derived from a wound site is large due to the length of the wound site, the depth of a wound, a technique, or the like, the exudate cannot be absorbed completely and may leak from the wound-covering material.

As described above, there has been a need for a wound-covering material that improves visibility of a wound site and has a high exudate absorbing rate and an appropriate exudate holding amount.

### Solution to Problem

The present inventors made intensive studies in order to solve the above problems. As a result, the present inventors have found, as one configuration of a wound-covering material, that a wound-covering material that can achieve both favorable visibility of a wound portion, and a high exudate absorbing rate and an appropriate exudate holding amount can be obtained by adopting a super absorbent pad in which a water-absorbing layer including a super absorbent fiber, a polypropylene fiber, and a polyethylene binder and a polyester nonwoven fabric are laminated and forming an opening passing through this super absorbent pad, and have completed the present invention.

That is, the present invention relates to
1. a wound-covering material including a super absorbent pad having a laminated structure of polyester nonwoven fabric layer/water-absorbing layer, in which the water-absorbing layer includes a super absorbent fiber, a polypropylene fiber, and a polyethylene binder, and the super absorbent pad has an opening passing therethrough.
   In addition, the present invention further provides the following embodiments.
2. The wound-covering material in which the water-absorbing layer includes a super absorbent fiber, a polypropylene fiber, and a polyethylene binder at a mass ratio of super absorbent fiber : polypropylene fiber : polyethylene binder = more than 30% by mass and less than 50% by mass : 40% by mass or more and less than 55% by mass : more than 10% by mass and less than 30% by mass (in which the total amount of the super absorbent fiber, the polypropylene fiber, and the polyethylene binder is 100% by mass) .
3. The wound-covering material in which the super absorbent pad has a basis weight of 200 to 400 g/m² and a thickness of 1.0 to 3.0 mm.
   The present invention also relates to
4. a wound-covering material including a super absorbent pad having a laminated structure of nonwoven fabric layer/water-absorbing layer, in which
   the super absorbent pad has a plurality of openings passing therethrough over an entire surface of the pad.
   Furthermore, the present invention further provides the following embodiments.
5. The wound-covering material in which each of the openings has a size of 10 to 500 mm².
6. The wound-covering material in which each of the openings has a triangular shape, a quadrangular shape, a rectangular shape, a parallelogrammical shape, a rhombic shape, a circular shape, an elliptical shape, a crescent shape, a horseshoe shape, a torus shape, or a combination thereof.
7. The wound-covering material in which the total area of the openings is 17% or more and less than 39% with respect to 100% of the total area of an upper surface of the super absorbent pad.

### Advantageous Effects of Invention

The present invention can provide a wound-covering material that can achieve both favorable visibility of a wound portion, and a high exudate absorbing rate and an appropriate exudate holding amount.

### Brief Description of Drawings

Fig. 1 is a cross-sectional view of a super absorbent pad in a preferable mode in a wound-covering material of the present invention.
Fig. 2 is a cross-sectional view of a wound-covering material in a preferable mode in the present invention.
Fig. 3 is a schematic view (front view) illustrating a mode of the shape of an opening in a super absorbent pad used in the present invention.
Fig. 4 is a schematic view (front view) illustrating a mode of the shape of an opening in the super absorbent pad used in the present invention.
Fig. 5 is a schematic view (front view) illustrating a mode of the super absorbent pad used in the present invention.
Fig. 6 is a schematic view (front view) of a super absorbent pad used in Example (Test Example 2).

### Description of Embodiments

A wound-covering material of the present invention includes a super absorbent pad having a laminated structure of polyester nonwoven fabric layer/water-absorbing layer, in which the water-absorbing layer includes a super absorbent fiber, a polypropylene fiber, and a polyethylene binder, and the super absorbent pad has an opening passing therethrough.

In addition, the wound-covering material of the present invention includes a super absorbent pad having a laminated structure of nonwoven fabric layer/water-absorbing layer, in which the super absorbent pad has a plurality of openings passing therethrough over an entire surface of the pad.

Hereinafter, each component of the wound-covering material of the present invention will be described in detail.

### [Super absorbent pad]

### <Polyester nonwoven fabric layer>

The polyester nonwoven fabric layer (hereinafter, also simply referred to as "nonwoven fabric layer") constituting the super absorbent pad plays a role of absorbing exudate derived from a wound site and allowing the exudate to permeate the water-absorbing layer described later and a role of holding the exudate that has been absorbed and gelled in the water-absorbing layer and preventing the exudate from returning to the wound site. Therefore, when the wound-covering material of the present invention is applied, the polyester nonwoven fabric layer in the super absorbent pad is located closer to a wound surface than the water-absorbing layer.

In addition, during a perforating process at the time of forming an opening described later or cutting the super absorbent pad, the polyester nonwoven fabric layer also plays a role of imparting shape retention so as not to impair processability thereof.

As the polyester nonwoven fabric layer, it is possible to use nonwoven fabrics manufactured by various processing methods such as a spun lace method, a spun bond method, a thermal bond method, a chemical bond method, an air laid method, a melt blown method, a needle punch method, and a stitch bond method.

Examples of the polyester include polyethylene terephthalate, polyethylene naphthalate, polymethylene terephthalate, and polybutylene terephthalate.

Above all, it is preferable to use a polyethylene terephthalate nonwoven fabric sheet manufactured by the spun bond method as the nonwoven fabric layer from viewpoints of liquid permeability, liquid absorbability, holdability of gelled exudate (prevention of return), shape retention and strength of the super absorbent pad during absorbing exudate, operability during manufacturing a wound-covering material or processing (perforating, cutting, or the like), and the like in addition to cost and biocompatibility. The adoption of the polyethylene terephthalate nonwoven fabric sheet manufactured by the spun bond method also leads to improvement of laminating strength when a net made of polyolefin is adopted in a surface layer described later.

The above nonwoven fabric layer can have an appropriate weight per area and thickness in consideration of liquid permeability, liquid absorbability, followability to the skin, and the like, and can have, for example, a weight per area of 10 to 30 g/m² and a thickness of 0.05 mm to 0.3 mm.

### <Water-absorbing layer>

The water-absorbing layer includes a super absorbent fiber, a polypropylene fiber, and a polyethylene binder.

The super absorbent fiber plays a role of gelling and holding exudate derived from a wound site by coming into contact with the exudate. Therefore, when a blending ratio of the super absorbent fiber is increased, a water absorption amount can be increased, but gel strength is low, which may lead to leakage of exudate (gel) derived from the super absorbent pad. Meanwhile, when the blending ratio of the super absorbent fiber is decreased, shape retention of the super absorbent pad is favorable, and gelled exudate can be prevented from leaking out, but a water absorption amount is reduced. As a result, this may lead to leakage of exudate that has not be completely absorbed by the super absorbent pad.

The polypropylene fiber plays a role of imparting shape retention of the water-absorbing layer. Furthermore, the polyethylene binder plays a role of improving adhesion between the super absorbent fiber and the polypropylene fiber and imparting shape retention of the water-absorbing layer. Therefore, when the blending ratios of the polypropylene fiber and the polyethylene binder are increased, the strength of exudate gel formed by the super absorbent fiber can be increased. However, this may lead to a decrease in a water absorption amount. Meanwhile, when the blending ratio of the polypropylene fiber and the polyethylene binder are decreased, a water absorption amount can be increased, but ability to hold exudate (gel) may be lacking. This may lead to deterioration of shape retention of the super absorbent pad and leakage of exudate (gel) .

As described above, the water absorption amount in the water-absorbing layer and the shape retention evaluated as the gel strength of the exudate are contradictory properties to each other. Therefore, in order to achieve both of these properties, it is necessary to select a blending ratio between the super absorbent fiber, and the polypropylene fiber and the polyethylene binder (hereinafter, hydrophobic material). The blending ratio (mass ratio) in the water-absorbing layer is preferably, for example, super absorbent fiber : hydrophobic material (polypropylene fiber and polyethylene binder) = more than 30% by mass to less than 50% by mass : more than 50% by mass to less than 70% by mass (the total amount of the super absorbent fiber and the hydrophobic material is 100% by mass) from this viewpoint.

Above all, by setting the blending ratio to super absorbent fiber : hydrophobic material = 31% by mass to 49% by mass : 51% by mass to 69% by mass, particularly preferably = 33% by mass to 43% by mass : 57% by mass to 67% by mass (the total amount of the super absorbent fiber and the hydrophobic material is 100% by mass), it is possible to obtain a water-absorbing layer and thus a super absorbent pad having an excellent balance between the water absorption amount and the gel strength.

A mass ratio between the polypropylene fiber and the polyethylene binder constituting the hydrophobic material is, for example, polypropylene fiber : polyethylene binder=5 : 1 to 1 : 3, preferably 3 : 2 to 1 : 1, for example, desirably 3 : 1 to 1 : 1 from a viewpoint of adhesion between fibers of the polyethylene binder described above (imparting shape retention),

Based on the above, for example, the blending ratio (mass ratio) among the super absorbent fiber, the polypropylene fiber, and the polyethylene binder is preferably super absorbent fiber : polypropylene fiber : polyethylene binder = more than 30% by mass and less than 50% by mass : 40% by mass or more and less than 55% by mass : more than 10% by mass and less than 30% by mass (in which the total amount of the super absorbent fiber, the polypropylene fiber, and the polyethylene binder is 100% by mass) .

The blending ratio (mass ratio) among the super absorbent fiber, the polypropylene fiber, and the polyethylene binder is more preferably super absorbent fiber : polypropylene fiber : polyethylene binder = 31% by mass to 49% by mass : 41% by mass to 50% by mass : 11% by mass to 19% by mass, and still more preferably 33% by mass to 40% by mass : 45% by mass to 50% by mass : 15% by mass to 17% by mass (in which the total amount of the super absorbent fiber, the polypropylene fiber, and the polyethylene binder is 100% by mass).

### <<Super absorbent fiber>>

Examples of the super absorbent fiber used in the present invention include fibers of super absorbent polymers described below. A composite fiber using two or more types of fibers of these super absorbent polymers may be used.

Examples of the super absorbent polymer include: a synthetic polymer such as a polyacrylic acid (salt)-based polymer, a polysulfonic acid (salt)-based polymer, a maleic anhydride (salt)-based polymer, a polyacrylamide-based polymer, a polyvinyl alcohol-based polymer, or a polyethylene oxide-based polymer; a natural product-derived polymer such as a polyaspartic acid (salt)-based polymer, a polyglutamic acid (salt)-based polymer, a polyalginic acid (salt)-based polymer, a starch-based polymer, or a cellulose-based polymer; cross-linked products thereof; and composites thereof (copolymers and the like). Above all, a sodium polyacrylate-based polymer is preferably used from a viewpoint of having excellent liquid absorbency.

### <<Polypropylene fiber>>

The polypropylene fiber plays a role of imparting the shape retention of the water-absorbing layer.

Note that the polypropylene fiber may be used in a form of a core-sheath type composite fiber in which the core is made of polypropylene and the shell is made of polyethylene, that is, a composite fiber coated with a polyethylene binder described later.

### <<Polyethylene binder>>

The polyethylene binder plays a role of improving adhesion between the super absorbent fiber and the polypropylene fiber and imparting shape retention of the water-absorbing layer.

### <<Manufacture of water-absorbing layer>>

A method for manufacturing the water-absorbing layer is not particularly limited, but for example, it is only required to mix the super absorbent fiber, the polypropylene fiber, and the polyethylene binder and to appropriately process the resulting mixture into a mat shape.

Note that by increasing the thickness of the water-absorbing layer or decreasing the density of the water-absorbing layer, the water absorption amount increases, and the gel strength (shape retention) of exudate decreases. As described above, by adjusting the thickness and density of the water-absorbing layer, the water absorption amount and the shape retention are changed. However, the water absorption amount and the shape retention as a wound-covering material can be changed depending on the types, thicknesses, basis weights, and densities of the polyester nonwoven fabric and other layers (surface layer, backing layer, and the like) described later, constituting the super absorbent pad. Furthermore, the water absorption amount and the shape retention can be changed depending on the pressure bonding strength (thickness, basis weight, or density) of each layer at the time of manufacturing the super absorbent pad.

Therefore, as an example, the thickness of the water-absorbing layer may be set to about 1.5 to 3.0 mm, and the basis weight thereof may be set to about 190 to 300 g/m² at the time of manufacturing the water-absorbing layer, and the thickness and the basis weight may be adjusted in the entire pad thickness and pad basis weight in a subsequent manufacture of the super absorbent pad.

The super absorbent pad can have a thickness of 1.0 to 3.0 mm and a basis weight of 200 to 400 g/m².

The thickness and basis weight in the laminated structure of polyester nonwoven fabric layer/water-absorbing layer can be set in a similar manner. However, in consideration of a case of disposing other layers described later, for example, the thickness can be set to about 1.5 to 3.0 mm, and the basis weight can be set to about 200 to 350 g/m² at the time of manufacturing the laminated structure. When other layers (surface layer, backing layer, and the like) described later are disposed, the pad thickness/pad basis weight of the entire super absorbent pad can be adjusted within the above numerical range by adjusting the thicknesses and basis weights of the other layers, a pressure bonding force at the time of disposing the other layers, and the like.

### <Other layers>

The super absorbent pad used for the wound-covering material of the present invention may further include a plurality of layers in addition to the laminated structure of the polyester nonwoven fabric layer/the water-absorbing layer.

For example, as a layer closer to a wound surface than the polyester nonwoven fabric layer, a surface layer in direct contact with a wound site or a backing layer backing the water-absorbing layer may be disposed. At this time, the surface layer can be laminated on the opposite side to a side on which the water-absorbing layer is disposed in the polyester nonwoven fabric layer, and the backing layer can be laminated on the opposite side to a side on which the polyester nonwoven fabric layer is disposed in the water-absorbing layer.

Between the surface layer and the polyester nonwoven fabric layer and between the water-absorbing layer and the backing layer, second and third water-absorbing layer and other layers (for example, second and third backing layers backing the second and third water-absorbing layers, respectively) may be disposed.

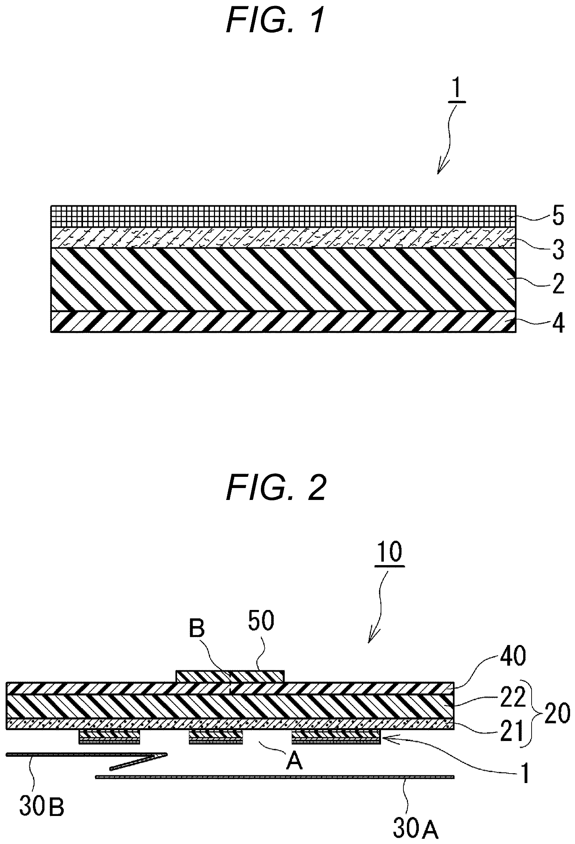

As an example, Fig. 1 illustrates a preferable mode of the super absorbent pad used in the wound-covering material of the present invention.

As illustrated in Fig. 1, a super absorbent pad 1 has the above-described laminated structure of a polyester nonwoven fabric layer 3/water-absorbing layer 2. A surface layer 5 is disposed on the opposite surface to a surface of the polyester nonwoven fabric layer 3 on which the water-absorbing layer 2 is disposed. A backing layer 4 is disposed on the opposite surface to a surface of the water-absorbing layer 2 on which the polyester nonwoven fabric layer 3 is disposed. Here, an upper side (surface layer 5) in Fig. 1 is a layer in direct contact with a wound site.

Note that although not illustrated in Fig. 1, by supplying a heat-fusible resin such as polyethylene powder between the surface layer 5 and the polyester nonwoven fabric layer 3, between the polyester nonwoven fabric layer 3 and the water-absorbing layer 2, and between the water-absorbing layer 2 and the backing layer 4, if desired, adhesion (heat-fusible property) of the two layers can be enhanced.

Although not illustrated in Fig. 1, the super absorbent pad 1 has an opening passing through the pad 1 (see Fig. 2 described later).

Hereinafter, a super absorbent pad having a laminated structure of surface layer 5/polyester nonwoven fabric layer 3/water-absorbing layer 2/backing layer 4 illustrated in Fig. 1 in a preferable mode of the present invention is referred to as "super absorbent pad A"
(Note that the "super absorbent pad" is a mode including the "super absorbent pad A").

A material or the like of the surface layer is not particularly limited as long as being a material that does not impair liquid absorbency of the polyester nonwoven fabric layer and the water-absorbing layer, and may have a porous structure (for example, a nonwoven fabric, a net-like shape, or punched) in order to facilitate liquid permeation.

For example, a net made of a polyolefin such as polyethylene or polypropylene can be used as the surface layer. Considering the liquid absorbency of the water-absorbing layer and followability to the skin (wound site), it is preferable to use a polyethylene net having a basis weight of about 10 to 30 g/m².

The "surface" here refers to a surface used as a surface which the exudate first permeates when the wound-covering material of the present invention is used.

The backing layer plays a role of holding exudate that has been absorbed and gelled in the water-absorbing layer to prevent the exudate from leaking out of the water-absorbing layer. In addition, during a perforating process at the time of forming an opening described later or cutting the super absorbent pad, the backing layer also plays a role of imparting shape retention so as not to impair processability thereof.

By further disposing the backing layer, when a pressure-sensitive adhesive film described later is adopted as a means for fixing the super absorbent pad to a wound site in the wound-covering material, an anchoring property to the pressure-sensitive adhesive film (pressure-sensitive adhesive layer described later) can be mentioned. In addition, since it is possible to prevent direct contact between the exudate that has been gelled in the water-absorbing layer and the pressure-sensitive adhesive film (pressure-sensitive adhesive layer), it is possible to suppress detachment of the super absorbent pad from the pressure-sensitive adhesive film, in which the detachment may occur due to contact between the gelled exudate and the pressure-sensitive adhesive film.

Note that presence of the backing layer may deteriorate moisture permeability of the super absorbent pad. However, in the present invention, desired moisture permeability can be ensured by forming an opening in the super absorbent pad.

The backing layer is not particularly limited as long as having the above properties. However, for example, a film or sheet made of a polyolefin such as polyethylene or polypropylene can be used. Considering the liquid absorbency of the water-absorbing layer and followability to the skin (wound site), for example, it is preferable to use a polyethylene film having a basis weight of 10 to 50 g/m².

### <Manufacture of super absorbent pad>

A method for manufacturing a super absorbent pad (for example, super absorbent pad A) used for the wound-covering material of the present invention is not particularly limited as long as the super absorbent pad has the laminated structure of polyester nonwoven fabric layer/water-absorbing layer.

For example, after the water-absorbing layer is manufactured, it is only required to laminate the water-absorbing layer and a polyester nonwoven fabric layer, and furthermore, if desired, to laminate other layers including a surface layer such as a polyolefin net and a backing layer such as a polyolefin film.

The water-absorbing layer and the polyester nonwoven fabric layer and, for example, the polyester nonwoven fabric layer and the surface layer, and the water-absorbing layer and the backing layer can be bonded (joined) to each other by heat fusion with a hot press roll. When the heat fusibility of each of these layers is insufficient, a heat fusible resin may be supplied between the respective layers to enhance adhesion (heat fusibility) of the two layers.

Note that the area of the super absorbent pad can be appropriately determined, and can be set, for example, within a range of 10 to 300 cm² in consideration of the area of a wound site or the like.

The shape of the super absorbent pad itself is not particularly limited, and can be selected according to a sticking location from among various shapes such as a quadrate (square, rectangle, or the like), a quadrangle (trapezoid, rhombus, or the like), a polygon, a circle, an ellipse, a semicircle, a triangle, a crescent, a combination thereof, and the like.

In the mode of the manufactured super absorbent pad, for example, the super absorbent pad A having the laminated structure of surface layer 5/polyester nonwoven fabric layer 3/water-absorbing layer 2/backing layer 4, the basis weight thereof can be 200 to 400 g/m², preferably 290 to 360 g/m², and the thickness can be 1.0 to 3.0 mm, preferably 1.2 to 2.0 mm.

### <Opening of super absorbent pad>

In the wound-covering material of the present invention, the super absorbent pad has an opening passing therethrough.

In the present invention, the opening is formed in a state where the opening passes through the super absorbent pad (a state where the opening is open all the time). The opening does not include, for example, a slit-shaped one, that is, a slit-shaped one that is expanded (opened) into an opening only by extending the wound-covering material but is closed while being as it is (the wound-covering material is not extended).

As described above, by forming an opening passing through the super absorbent pad, a wound portion can be visually recognized from the opening without peeling off the wound-covering material, a healing condition of a wound surface and signs of infectious diseases can be observed, and a timing of changing the wound-covering material and execution or review of a wound treatment plan can be judged.

By processing the opening, the flexibility of the super absorbent pad can be improved, and the surface area around the opening is increased due to the opening, which leads to an increase in a water absorption rate.

The size and shape of the opening can be appropriately selected such that a wound portion can be sufficiently confirmed, the deformation amount of the opening is small during manufacture of the wound-covering material, a suitable mechanical strength as the super absorbent pad of the wound-covering material is maintained, a sufficient water absorption property as the wound-covering material is ensured, and such a strength and shape retention that the shape of the opening is maintained can be ensured when the opening absorbs exudate.

For example, each of the openings has a size of 10 to 500 mm², preferably a size of 50 to 400 mm². The total area of the openings can be 17% or more and less than 39%, and preferably 20% to 33% (also referred to as open porosity or open pore area) with respect to the total area (100%) of an upper surface of the super absorbent pad. The openings may be formed at a plurality of locations in one super absorbent pad disposed in one wound-covering material. In this case, the total area of the plurality of openings only needs to be within the above numerical range.

The shape of each of the openings is not particularly limited, and may be a polygon such as a triangle, a quadrangle, a rectangle, a parallelogram, or a rhombus, a shape including an arc, such as a circle, an ellipse, an oval, a semicircle, a crescent, a horseshoe, or a torus, or a combination thereof. For example, the shape of each of the openings may be a shape having a longitudinal direction thereof inclined with respect to the longitudinal direction of the super absorbent pad from the above-described viewpoints of ensuring the visibility of the openings, the small deformation amount of the openings, and maintaining the strength of the super absorbent pad.

Note that as for the openings, after the super absorbent pad is cut into a predetermined size in one wound-covering material, a process of forming an opening passing through the super absorbent pad may be performed. Alternatively, after a process of forming an opening passing through the super absorbent pad is performed, the super absorbent pad may be cut into a predetermined size in one wound-covering material.

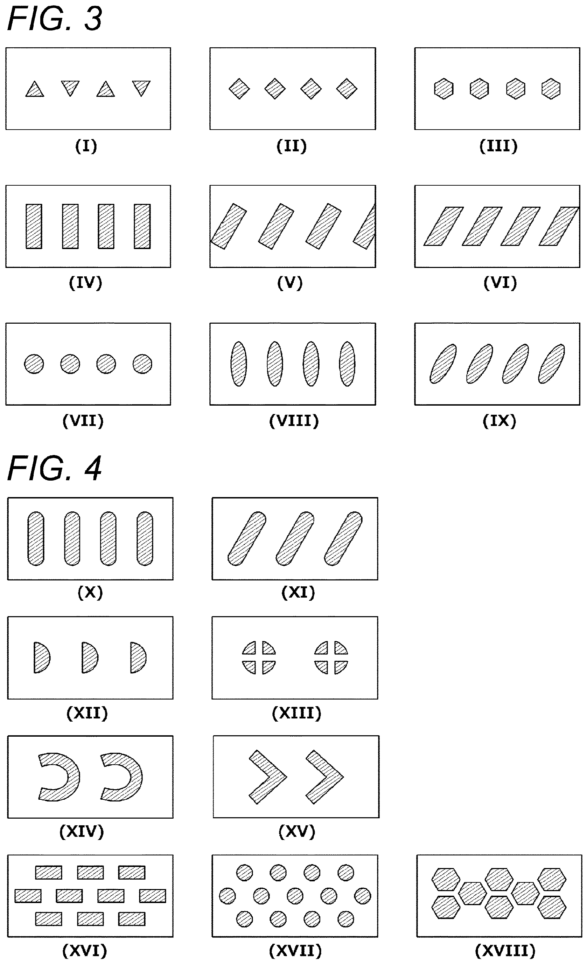

For example, various modes of the shapes of the above openings are illustrated in Figs. 3 and 4. (I) to (IX) of Fig. 3 and (X) to (XVIII) of Fig. 4 each illustrate a schematic view (front view) of a super absorbent pad having an opening passing therethrough. The hatched portions in the drawings indicate the openings. Note that the number and arrangement (vertical and horizontal directions) of the openings illustrated in the drawings are not limited thereto.

As illustrated in Fig. 3, examples of the shapes of the openings include (I) triangle, (II) quadrangle (rhombus), (III) hexagon, (IV) rectangle, (X) rectangle (inclined), (XI) parallelogram, (XII) circle, (XIII) ellipse, and (IX) ellipse (inclined). As illustrated in Fig. 4, examples of the shapes of the openings include (X) oval, (XI) oval (inclined), (XII) semicircle, (XIII) quarter circle, (XIV) horseshoe shape, and (XV) L-shape. As illustrated in (XIII) and (XVI) to (XVIII) of Fig. 4, the openings can be formed so as to be arranged vertically and horizontally.

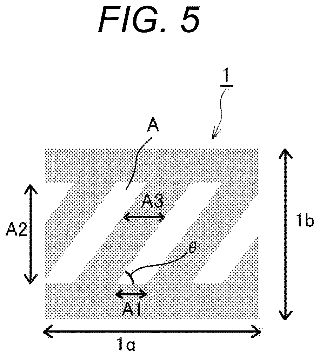

Above all, a preferable mode of the openings is illustrated in Fig. 5. Fig. 5 is a schematic view (front view) of a super absorbent pad. In the rectangular super absorbent pad 1, the openings A are formed at a plurality of locations in a form inclined by an angle θ with respect to a longitudinal width 1a of the pad 1 (parallelogram).

In this way, for example, by forming the opening in the shape in which an axial direction of the opening is inclined with respect to one axial direction of the super absorbent pad, in a case where the above-described process of forming the opening in the super absorbent pad is performed and then the super absorbent pad is cut into a predetermined size, the strength of the pad can be easily maintained advantageously during the series of processes. In addition, the shape of the inclined opening makes the stiffness of the pad high. Therefore, the pad is unlikely to be deformed advantageously during processing such as cutting the pad or manufacturing the wound-covering material. Furthermore, dust (powder) is unlikely to be generated advantageously when the pad is cut after formation of the opening. In addition, contact of the super absorbent pad with respect to a wound site can be uniform. That is, contact between a wound surface and an exudate absorption surface (so-called unopened portion of the super absorbent pad) can be uniform.

Furthermore, for example, as illustrated in Fig. 5, in a case of a rectangular super absorbent pad, by making a widthwise edge (A1) of the opening parallel to a longitudinal width (1a) of the super absorbent pad, in short, by forming an edge of the opening so as to be parallel to an edge of the super absorbent pad, when the wound-covering material is applied to a wound site, it is easy to grasp the position of the opening with respect to the wound site, which assists application to an appropriate site.

### [Wound-covering material]

The structure of the wound-covering material of the present invention is not particularly limited as long as including the above-described super absorbent pad having an opening passing therethrough.

For example, a means for fixing the super absorbent pad to a wound portion (for example, a pressure-sensitive adhesive film including a support layer and a pressure-sensitive adhesive layer), a release material that protects an application surface (the surface layer or the like) of the super absorbent pad (prevents attachment of dust and the like) until just before application and is peeled off at the time of application (release sheet or release paper), a carrier film (also referred to as backing film) that improves handleability of the wound-covering material, or an intake tape that assists release of the carrier film may be disposed.

Note that only the super absorbent pad and the pressure-sensitive adhesive film which is a means for fixing the super absorbent pad in a preferable mode are finally applied to (stuck on) a wound site and the skin around the wound site. However, here, a material also including the release material, the carrier film, and the intake tape, which are peeled off at the time of application, is referred to as "wound-covering material".

Hereinafter, as an example of a preferable mode of the wound-covering material of the present invention, a cross-sectional view thereof is illustrated in Fig. 2.

As illustrated in Fig. 2, as an example of a preferable mode, a wound-covering material 10 includes, in addition to the super absorbent pad 1 (see Fig. 1), a pressure-sensitive adhesive film 20 as a means for fixing the super absorbent pad 1 to the skin around a wound portion (the super absorbent pad 1 and the pressure-sensitive adhesive film 20 serve as a wound-covering material after application in a narrow sense). The opening A is formed in the super absorbent pad 1 so as to pass therethrough in a lamination direction of the pad. The pressure-sensitive adhesive film 20 includes at least a pressure-sensitive adhesive layer 21 and a support layer 22. Note that the super absorbent pad 1 includes a backing layer 4 (the reference numeral is not illustrated in the drawing) of the pad 1 stuck on the pressure-sensitive adhesive layer 21 of the pressure-sensitive adhesive film 20. In addition, the super absorbent pad 1 includes a release material 30 (30A, 30B) that protects an application surface of the super absorbent pad 1 (surface layer 5, the reference numeral is not illustrated in the figure) and the pressure-sensitive adhesive layer 21 of the pressure-sensitive adhesive film 20 until just before application, a carrier film 40 on the opposite surface to a side on which the super absorbent pad 1 of the pressure-sensitive adhesive film 20 is disposed, and an intake tape 50 on the carrier film 40. In the present mode, the release material 30 includes two sheets of a sheet 30A having a larger area and a sheet 30B having a smaller area. The carrier film 40 and the intake tape 50 preferably have a half cut B. By cutting the carrier film 40 and the intake tape 50 at this half cut B, the carrier film 40 near the half cut B (and the intake tape 50) easily becomes pre-lifted, and the carrier film 40 (and the intake tape 50) is easily peeled off from the pressure-sensitive adhesive film 20.

### <Pressure-sensitive adhesive film>

The pressure-sensitive adhesive film has a laminated structure of at least two layers including a support layer and a pressure-sensitive adhesive layer disposed on a surface thereof.

The pressure-sensitive adhesive film plays a role of fixing the super absorbent pad to a wound site, and is stuck on a backing layer of the super absorbent pad in a preferable mode, and is not stuck even on a release material described later before application.

For this reason, the pressure-sensitive adhesive film preferably has an area slightly larger than the area of the super absorbent pad, and preferably has a size formed by being stuck on a release material described later. For example, the area of the super absorbent pad with respect to the area of the pressure-sensitive adhesive film can be set to 25% or more and less than 50%. When the area of the super absorbent pad is too small with respect to the area of the pressure-sensitive adhesive film (for example, less than 20%), a sufficient water absorption property cannot be necessarily obtained, and in addition, skin irritation may occur because the area of the pressure-sensitive adhesive layer of the pressure-sensitive adhesive film stuck on the skin around a wound site is large. When the area of the super absorbent pad is too large with respect to the area of the pressure-sensitive adhesive film (for example, more than 50%), a sufficient pressure-sensitive adhesive force for fixing the super absorbent pad to a wound site cannot be obtained because the area of the pressure-sensitive adhesive layer of the pressure-sensitive adhesive film stuck on the skin around the wound site is small. This may lead to peeling.

Note that the position of the pressure-sensitive adhesive film stuck on the super absorbent pad is not particularly limited. For example, the central portion of the pressure-sensitive adhesive film may be stuck on the super absorbent pad, or an outer side of the central portion of the pressure-sensitive adhesive film may be stuck on the super absorbent pad.

The support layer is not particularly limited as long as having appropriate stretchability, flexibility, and strength, and being able to ensure transparency that allows a wound portion to be visually recognized from an opening of the super absorbent pad. However, the support layer particularly preferably has appropriate moisture permeability and a fungal barrier property. Examples of the support layer include a film and a form made of a polyolefin, a polyurethane, a polyester, or a polyacrylic acid. Above all, a polyurethane film is preferable from the above viewpoint.

When the support layer is a polyurethane film, a polyester or polyether-based polyurethane is preferably used, and is also desirably a film that does not easily swell when being in contact with water or another liquid such as a treatment liquid. A polyether-based polyurethane film is preferably used from such a viewpoint.

The thickness of this polyurethane film can be in a range of usually 1 to 100 µm, preferably 8 to 50 µm, more preferably 10 to 40 µm.

When the thickness of the polyurethane film is less than 1 µm, in addition to difficulty in film formation, the strength as the support layer is insufficient, and the support layer may be damaged (broken) when the wound-covering material is stuck on/peeled off from an adherend (wound portion). When the thickness of the polyurethane film is too large, even if the thickness of the entire wound-covering material is small, it is difficult to closely attach the wound-covering material along a skin surface with fine unevenness such as skin grooves, and followability to the adherend deteriorates. In addition, a trigger for peeling is easily formed around the wound-covering material, which may lead to peeling from the adherend, an increase in discomfort while the wound-covering material is stuck, and a risk of liquid leakage from the super absorbent pad.

The support layer of the pressure-sensitive adhesive film has a moisture permeability of, for example, 1,000 g/m²•hr or more, preferably 2,000 g/m²•24 hr or more. An upper limit of the moisture permeability of the support layer is not particularly limited, but is usually about 10,000 g/m²•24 hr or less, and preferably about 8,000 g/m²•24 hr or less. The urethane film support having such a moisture permeability is useful as a dressing material, is known per se (JP 7-231910 A and the like), and is commercially available.

The pressure-sensitive adhesive layer is formed of a pressure-sensitive adhesive containing a synthetic resin. The synthetic resin contained in the pressure-sensitive adhesive is not particularly limited as long as having low skin irritation, and examples thereof include a pressure-sensitive adhesive containing an acrylic resin, a urethane-based resin, a silicone-based resin, a rubber-based resin, a polyvinyl alcohol-based resin, a polyamide-based resin, a polyvinyl acetate-based resin, or another synthetic resin. Among these pressure-sensitive adhesives, an acrylic pressure-sensitive adhesive made of an acrylic resin; a urethane-based pressure-sensitive adhesive such as a polyether-based polyurethane or a polyester-based polyurethane; a silicone-based pressure-sensitive adhesive such as an organopolysiloxane or an alkylarylpolysiloxane; and a rubber-based adhesive such as polyisobutylene, a butyl rubber, a styrene-butadiene copolymer (SBR), a styrene-isoprene-styrene block copolymer (SIS), a styrene-butadiene-styrene block copolymer (SBS), a styrene-ethylene-butylene-styrene block copolymer (SEBS), a styrene-ethylene-propylene-styrene block copolymer (SEPS), or a hydrogenated product of SBR are preferably used. Above all, a urethane-based pressure-sensitive adhesive is particularly preferable from a viewpoint of adhesion and skin irritation.

The thickness of the pressure-sensitive adhesive layer can be appropriately selected in a range of usually 5 to 180 µm, preferably 10 to 150 µm.

For example, in a pressure-sensitive adhesive film in which a urethane film is adopted as a support layer and a polyurethane pressure-sensitive adhesive layer is disposed thereon, the moisture permeability thereof can be, for example, 2,000 to 5,000 g/m²•24 hr, and preferably 3,000 to 4,000 g/m²•24 hr.

### <Release material>

The wound-covering material of the present invention may further include a release material. The release material is usually laminated on a layer in direct contact with a wound site in the super absorbent pad (for example, surface layer), and on the pressure-sensitive adhesive layer of the pressure-sensitive adhesive film that can be present around the super absorbent pad, and can play a role of protecting these layers to prevent attachment of dust and the like until just before application.

As the above release material, those conventionally used in the technical field of a wound-covering material or a sticking material can be used. Examples of the release material include: a plastic film such as a polyester (polyethylene terephthalate, polybutylene terephthalate, polyethylene naphthalate, or the like), polypropylene (unstretched polypropylene, stretched polypropylene, or the like), polyethylene, polyurethane, polyvinyl chloride, or polystyrene; paper such as fine paper, glassine paper, parchment paper, or craft paper and synthetic paper; release-processed paper obtained by coating the plastic film, the paper or synthetic paper, a synthetic fiber, or the like with a release agent having a release property, such as a silicone resin or a fluorocarbon resin; an aluminum foil; and a colorless or colored sheet such as laminated paper obtained by laminating these various films and sheets or laminated release paper obtained by coating the laminated paper with a release agent. Note that the release material can have unevenness so as to be easily taken out from a packaging material.

The thickness of the release material is not particularly limited, but is usually 10 µm to 1 mm, for example, 20 µm to 500 µm, and preferably 40 µm to 200 µm.

The shape of the release material can be a quadrate, a rectangle, a circle, or the like, and can be a shape with rounded corners if desired. The size of the release material can be the same as or slightly larger than the size of the pressure-sensitive adhesive film in the wound-covering material. The release material can be formed of one sheet or a plurality of divided sheets. A cut thereof may be formed by a straight line, a wavy line, or a sewing machine line, and some parts of the release material may overlap each other.

### <Carrier film>

In the pressure-sensitive adhesive film, when the thickness of the support layer is extremely thin, for example, about 1 µm to 30 µm, disposition of a releasable carrier film on the opposite surface to the pressure-sensitive adhesive layer formed on the support layer is preferable because handleability as the wound-covering material is improved.

As the carrier film, it is preferable to use various films made of various thermoplastic resins such as polyester, polyurethane, polyethylene, polypropylene, an ionomer, polyamide, polyvinyl chloride, polyvinylidene chloride, an ethylene vinyl acetate copolymer, thermoplastic polyester, and polytetrafluoroethylene. As the carrier film, a film obtained by laminating the above various films on paper may be used. Above all, a polypropylene film is preferably used from a viewpoint that handleability as the wound-covering material can be preferable.

In order to achieve a purpose of improving handleability as the wound-covering material, the carrier film desirably has a large thickness or is made of a stiff material. The thickness of the carrier film is usually 10 µm to 500 µm, and preferably 20 µm to 250 µm. When the thickness of the carrier film is less than 10 µm, the support of the pressure-sensitive adhesive film and the carrier film do not come into close contact. When the thickness of the carrier film exceeds 500 µm, adhesion with the support of the pressure-sensitive adhesive film is sufficient to improve operability, but the rigidity of the carrier film is too high. For example, when the release material is peeled off at the time of use and the super absorbent pad is attached to a wound site, and the pressure-sensitive adhesive layer of the pressure-sensitive adhesive film is stuck on the skin around the wound site, followability to the wound site or the skin around the wound site is lacking, and a sticking property is insufficient.

The carrier film may have a half cut in a longitudinal direction, a lateral direction, or an oblique direction of the film. The half cut can be formed so as to leave a small connecting portion, instead of separating the entire carrier film. Due to the half cut formed on the carrier film, by cutting the wound-covering material at this half cut at the time of application of the wound-covering material, the carrier film can be more easily peeled off from the pressure-sensitive adhesive film.

### <Intake tape>

In the wound-covering material of the present invention, an intake tape may be stuck on the carrier film, that is, on a surface of the carrier film opposite to a side on which the pressure-sensitive adhesive film is disposed. The intake tape is preferably stuck on a part of the surface of the carrier film, and more preferably has the above-described half cut together with the carrier film.

By sticking the intake tape on the carrier film, the thickness (stiffness) of the stuck portion increases. Therefore, when a half cut is formed so as to pass through the intake tape and the carrier film, by cutting the wound-covering material at this half cut at the time of application of the wound-covering material, the carrier film (and the intake tape) around the half cut can be more easily peeled off. It is also possible to print a symbol or a character serving as a peeling mark on the intake tape.

The intake tape has a laminated structure of at least two layers including a tape support and a tape pressure-sensitive adhesive layer disposed on a surface of the tape support. As the tape support, various films listed in the support layer of <Pressure-sensitive adhesive film> or <Carrier film> described above can be appropriately used. As the tape pressure-sensitive adhesive layer, various pressure-sensitive adhesives listed in the pressure-sensitive adhesive layer of <Pressure-sensitive adhesive film> described above can be appropriately used.

As an example of a method for applying the wound-covering material of the present invention, an application example of the above-described wound-covering material 10 illustrated in Fig. 2 will be described below. First, out of the release materials 30, the release material 30A having a larger area is peeled off to expose a part of the super absorbent pad 1 and the pressure-sensitive adhesive layer 21 of the pressure-sensitive adhesive film 20. Then, the super absorbent pad 1 is applied to a wound site (wound surface), the remaining release material 30B is peeled off, and the pressure-sensitive adhesive layer 21 of the pressure-sensitive adhesive film 20 is stuck on the skin around the wound site to fix the super absorbent pad 1 to the wound site. Then, the carrier film 40 and the intake tape 50 are cut at the half cut B formed on the carrier film 40 and the intake tape 50. The carrier film 40 and the intake tape 50 are peeled off from the pressure-sensitive adhesive film 20 starting from the cut location, and the super absorbent pad 1 and the pressure-sensitive adhesive film 20 are brought into close contact with the wound site and a site around the wound site.

The wound-covering material thus applied can absorb exudate and the like derived from the wound site in the super absorbent pad, and can gel and hold (hold moisture of) the exudate. In addition, by forming an opening passing through the super absorbent pad, a state of a wound portion can be confirmed, and moisture permeability can be secured.

As described above, in the wound-covering material of the present invention, it can be expected to maintain a moist environment of a wound to achieve moist healing by visibility and high moisture permeability imparted by an opening of the super absorbent pad and absorbing and holding exudate in a portion other than the opening.

### Examples

Hereinafter, the present invention will be described in more detail with reference to Examples, but the present invention is not limited to these Examples.

### [Examples 1 to 7 and Comparative Examples 1 to 7]

### [Method for manufacturing super absorbent pad]

Polyethylene powder was attached onto a polyester nonwoven fabric layer formed by a spun bond method in order to enhance a heat fusion property. On the polyethylene powder, fibers obtained by mixing a super absorbent fiber (SAF), a polypropylene fiber (PP), and a polyethylene binder (PE), constituting a water-absorbing layer, at a ratio illustrated in Table 1 were accumulated, and the fibers were attached to each other by heat fusion in an oven to form a water-absorbing layer and a laminated structure of the water-absorbing layer/polyester nonwoven fabric layer at the same time.

Subsequently, a polyolefin film (backing layer) coated with a hot melt adhesive was stuck on a surface of the water-absorbing layer opposite to a surface on which the polyester nonwoven fabric layer was disposed.

Then, a polyethylene net (surface layer) was heatfused onto the polyester nonwoven fabric layer. Thereafter, the resulting product was punched into a predetermined size (two types of sizes, see the following) so as to have a parallelogram opening to prepare a super absorbent pad (super absorbent pad A). Note that among evaluations of properties of the super absorbent pad described later, a pad having no opening was used for evaluation of water absorption amount and gel strength.

As illustrated in Table 1, by changing the composition of the water-absorbing layer and the composition of the nonwoven fabric layer, the super absorbent pads in Examples 1 to 7 and Comparative Examples 1 to 7 were manufactured in which the compositions of the layers were different. Note that Table 1 illustrates the basis weight and the total pad thicknesses of each of the laminated structure after the backing layer and the surface layer are disposed (that is, super absorbent pad A: polyolefin film (backing layer)/water-absorbing layer/nonwoven fabric layer/polyethylene net (surface layer)), and the laminated structure before the backing layer and the surface layer are disposed (water-absorbing layer/nonwoven fabric layer).

### [Method for manufacturing wound-covering material]

100 parts of a polyurethane-based pressure-sensitive adhesive and 2 parts of a cross-linking agent (Coronate L, Tosoh Co., Ltd. [former Nippon Polyurethane Industry Co., Ltd.]) were uniformly mixed and then defoamed. The resulting product was applied onto a polyester film (release body, thickness 50 µm) that had been subjected to a release treatment using a knife coater so as to have a dry film thickness of 25 µm, and then cured and dried at 120°C for three minutes to form a pressure-sensitive adhesive layer.

On the obtained pressure-sensitive adhesive layer, a laminate of an ether-based urethane resin film substrate having a thickness of 20 µm (moisture permeability 3,300 g/m²•day), which is a support layer, and a polypropylene film having a thickness of 40 µm, which is a carrier film, was stuck such that a surface of the support layer faced the pressure-sensitive adhesive layer. Thereafter, the resulting product was stored in an atmosphere of 50°C in a hot air drier for five days to complete a cross-linking reaction of the pressure-sensitive adhesive, thus preparing a pressure-sensitive adhesive film including a carrier film stuck on the release body.

The release body of the pressure-sensitive adhesive film was removed. A polyolefin film surface, which is a backing layer of the super absorbent pad cut into a predetermined size (super absorbent pad in each of Examples 1 to 7 and Comparative Examples 1 to 7 in which the compositions of the layers were different), was stuck on the pressure-sensitive adhesive layer of the pressure-sensitive adhesive film. A release material (silicone-treated poly-laminate fine paper, thickness 110 µm) was stuck on the pressure-sensitive adhesive layer of the pressure-sensitive adhesive film and the super absorbent pad stuck on the pressure-sensitive adhesive layer so as to cover the pressure-sensitive adhesive layer and the super absorbent pad. The resulting product was punched into a specified size to prepare a wound-covering material including the release material.

### <Super absorbent pad: Manufacturing Examples 1 and 2 having different shapes (sizes)>

For each of Examples 1 to 7 and Comparative Examples 1 to 7, two types of super absorbent pads (1) and (2) having the following sizes and openings were manufactured.

### [Manufacturing Example 1: super absorbent pad (1)]

- Pad size: width in short direction (Fig. 5: 1b) 30 mm
   width in longitudinal direction (Fig. 5: 1a) 65 mm
- Pad structure: Examples 1 to 7 and Comparative Examples 1 to 7 described above (see Table 1)
- Opening shape: length (Fig. 5: A1) 5 mm x height (Fig. 5: A2) 12 mm
- Opening inclination angle (Fig. 5: θ): 40°
- Opening interval (Fig. 5: A3): 6 mm
- Area ratio of openings in super absorbent pad: 20%

### [Manufacturing Example 2: super absorbent pad (2)]

- Pad size: width in short direction (Fig. 5: 1b) 50 mm
   width in longitudinal direction (Fig. 5: 1a) 100 mm
- Pad structure: Examples 1 to 7 and Comparative Examples 1 to 7 described above (see Table 1)
- Opening shape: length (Fig. 5: A1) 10 mm x height (Fig. 5: A2) 30 mm
- Opening inclination angle (Fig. 5: θ): 40°
- Opening interval (Fig. 5: A3): 12 mm
- Area ratio of opening in super absorbent pad: 27%

### <Wound-covering material: Manufacturing Examples 3 and 4 having different shapes (sizes)>

Using the super absorbent pads (1) obtained in the above Manufacturing Example 1 [14 types in total of Examples 1 to 7 and Comparative Examples 1 to 7 in which the compositions of the layers were different], and the super absorbent pads (2) obtained in Manufacturing Example 2 [14 types in total of Examples 1 to 7 and Comparative Examples 1 to 7 in which the compositions of the layers were different], a wound-covering material (1) and a wound-covering material (2) having the following sizes were manufactured. Note that, in any of the wound-covering materials, the pad was set so as to be disposed substantially in the center of the pressure-sensitive adhesive film.

### [Manufacturing Example 3: wound-covering material (1)]

- Pad used: super absorbent pad (1)
- Wound-covering material (pressure-sensitive adhesive film) size: width in short direction 60 mm
   width in longitudinal direction 100 mm

### [Manufacturing Example 4: wound-covering material (2)]

- Pad used: super absorbent pad (2)
- Wound-covering material (pressure-sensitive adhesive film) size: width in short direction 90 mm
   width in longitudinal direction 150 mm

### [Evaluation of property of super absorbent pad]

According to the following procedure, the water absorption amount of each of the super absorbent pads in Examples 1 to 7 and Comparative Examples 1 to 7, gel strength when each of the pads was caused to absorb water, whether or not paper dust was generated during pad processing, and suitability for perforating and cutting were evaluated.

Note that in Examples, the evaluation was made with the super absorbent pad in which a polyethylene net as a surface layer and a polyolefin film as a backing layer were laminated. It has been confirmed that the surface layer and the backing layer do not affect various characteristics such as a water absorption amount.

### [1] Evaluation of water absorption amount

### <Evaluation procedure>

Each of the manufactured super absorbent pads A (having no opening, including a surface layer and a backing layer) was cut into a size of 50 mm × 50 mm, was caused to absorb a saline solution adjusted to 0.9% by mass, and was allowed to stand for two hours. The mass of each of the super absorbent pads was measured before water absorption and after being allowed to stand for two hours after water absorption, and the amount of saline solution absorbed by each of the super absorbent pads was calculated in terms of mass per unit area.

For a market product (wound-covering material having an opening), a release material portion was peeled off, and the resulting product was placed such that a pad portion faced upward. Thereafter, the pad was caused to absorb the saline solution, and was allowed to stand for two hours. Thereafter, excess water that had not been absorbed was wiped off. A water absorption amount per unit area was calculated by a similar procedure (0.16 g/cm²). Considering that the area of the opening of the market product was about 30% of the whole area, the water absorption amount of the market product having no opening was estimated to be 0.23 g/cm², and this value was used as a target value. Then, whether or not the water absorption amount per unit area of the super absorbent pad (having no opening) of the present invention satisfied the above target value was used as a criterion for judgment.

The number of tests n was set to three in each case, and evaluation was made based on an average value (Table 1 also illustrates a range of a difference between a maximum value and a minimum value).

### <Evaluation criterion>

○: 0.23 g/cm² or more
×: less than 0.23 g/cm²

### [2] Evaluation of gel strength

### <Evaluation procedure>

Each of the manufactured super absorbent pads (having no opening, including a polyethylene net as a surface layer) was cut into a size of 50 mm × 50 mm, was caused to absorb a saline solution adjusted to 0.9% by mass, and was allowed to stand for two hours. After each of the super absorbent pads was allowed to stand, a weight of 1,000 g/50 mmφ was placed on each of the super absorbent pads, and a gel exudation area was evaluated.

### <Evaluation criterion>

○: Gel exudation area is less than 5% with respect to pad area
×: Gel exudation area is 5% or more and less than 10% with respect to pad area
××: Gel exudation area is 10% or more with respect to pad area

### [3] Evaluation of paper dust generation

### <Evaluation procedure>

For the super absorbent pads (1) manufactured in Manufacturing Example 1 having different sizes [Examples 1 to 7 and Comparative Examples 1 to 7 in which the compositions of the layers were different], whether or not paper dust was generated during an opening punching process and a pad planting process to a pressure-sensitive adhesive film was evaluated.

### <Evaluation criterion>

○: No paper dust is generated during opening punching process and pad planting process
×: Paper dust is generated during opening punching process and pad planting process

### [4] Suitability for perforating and cutting

### <Evaluation procedure>

For the super absorbent pads (1) having different sizes, manufactured in Manufacturing Example 1 [Examples 1 to 7 and Comparative Examples 1 to 7 in which the compositions of the layers were different], whether or not a perforating (opening) process and a cutting process were favorable was evaluated by size stability.

Regarding suitability for a perforating process, a deviation length of an interval (pitch) between openings was measured.

Regarding suitability for a cutting process, when each of the pads was cut into a prescribed size, a deviation length from the size was measured.

### <Evaluation criterion>

×: A deviation length of a pitch is 1.0 mm or more, and
a deviation length from the cutting size is 1.5 mm or more
Δ: A deviation length of a pitch is 0.5 mm or more and less than 1.0 mm, and
a deviation length from the cutting size is 1.1 mm or more and less than 1.5 mm
○: A deviation length of a pitch is less than 0.5 mm, and
a deviation length from the cutting size is less than 1.1 mm

Results obtained above are illustrated in Table 1.

### [Table 1]

**[Table 1]**

| | | Composition of water-absorbing layer*¹ (% by mass) | | | Water absorption amount*³ | | | Gel strength*4 | Paper dust*⁵ | Suitability for perforating and cutting*⁶ | Super absorbent pad A (incl uding surface layer and backing layer) | | Nonwoven fabric layer/water-absorbing layer (not including surface layer or backing layer) | | Nonwoven fabric layer*² |
|---|---|---|---|---|---|---|---|---|---|---|---|---|---|---|---|
| | | | | | Average value | Range | Evaluation*³ | | | | | | | | |
| | | | | | | | | | | | Basis weight | Total pad thickness | Basis weight | Total pad thickness | |
| | | SAF | PP | PE | g/cm² | | | | | | g/cm² | mm | g/cm² | mm | |
| Example | 1 | 36 | 48 | 16 | 0.286 | 0.004 | ○ | ○ | ○ | ○ | 347.1 | 1.66 | 265 | 2.3 | PET-SB |
| | 2 | 36 | 48 | 16 | 0.267 | 0.009 | ○ | ○ | ○ | ○ | 352.3 | 1.48 | 265 | 1.9 | PET-SB |
| | 3 | 36 | 48 | 16 | 0.345 | 0.002 | ○ | ○ | ○ | ○ | 348.0 | 1.85 | 265 | 2.6 | PET-SB |
| | 4 | 36 | 48 | 16 | 0.295 | 0.006 | ○ | ○ | ○ | ○ | 294.4 | 1.29 | 220 | 2.2 | PET-SB |
| | 5 | 33 | 50 | 17 | 0.301 | 0.014 | ○ | ○ | ○ | ○ | 336.7 | 1.58 | 265 | 2.2 | PET-SB |
| | 6 | 40 | 45 | 15 | 0.288 | 0.009 | ○ | ○ | ○ | ○ | 351.5 | 1.67 | 265 | 2.2 | PET-SB |
| | 7 | 40 | 45 | 15 | 0.327 | 0.011 | ○ | ○ | ○ | ○ | 360.0 | 1.63 | 280 | 2.3 | PET-SB |
| Comparative Example | 1 | 30 | 50 | 20 | 0.183 | 0.007 | × | ○ | ○ | Δ | 300.0 | 1.41 | 240 | 2.1 | PP-AT |
| | 2 | 50 | 40 | 10 | 0.366 | 0.026 | ○ | ×× | ○ | Δ | 271.0 | 1.48 | 215 | 2.3 | PP-AT |
| | 3 | 40 | 50 | 10 | 0.287 | 0.014 | ○ | × | ○ | ○ | 287.5 | 1.38 | 240 | 1.9 | PET-SB |
| | 4 | 40 | 50 | 10 | 0.306 | 0.035 | ○ | × | ○ | × | 277.2 | 1.27 | 240 | 1.9 | Tissue |
| | 5 | 40 | 45 | 15 | 0.307 | 0.009 | ○ | ○ | ○ | Δ | 346.8 | 1.61 | 265 | 2.3 | PP-AT |
| | 6 | 33 | 50 | 17 | 0.286 | 0.009 | ○ | ○ | ○ | Δ | 342.5 | 1.51 | 265 | 2.2 | rayon-SL |
| | 7 | 50 | 40*¹ | 10 | 0.462 | 0.055 | ○ | × | ×× | Δ | 219.9 | 1.37 | 165 | - | PP-AT |

| | | | | | | | | | | | | | | | |
|---|---|---|---|---|---|---|---|---|---|---|---|---|---|---|---|
| *1: In Comparative Example 7, pulp was used in place of PP. *2: Nonwoven fabric layer PET-SB: PET spun bond nonwoven fabric, PP-AT: PP air through nonwoven fabric, rayon-SL: rayon spun lace nonwoven fabric Note that in Comparative Example 4, tissue (paper) was used in place of nonwoven fabric. *3: Water absorption amount evaluation o: 0.23 g/cm² or more ×: less than 0.23 g/cm² *4: Gel strength evaluation ○: Gel exudation area is less than 5% of pad area ×: Gel exudation area is 5% or more and less than 10% of pad area ××: Gel exudation area is 10% or more of pad area *5: Paper dust evaluation o: No paper dust is generated during opening punching ××: Paper dust is generated during punching *6: Suitability for perforating and cutting evaluation o: Deviation length of pitch is less than 0.5 mm, and deviation length from cutting size is less than 1.1 mm Δ: Deviation length of pitch is 0.5 mm or more and less than 1.0 mm, and deviation length from cutting size is 1.1 mm or more and less than 1.5 mm ×: Deviation length of pitch is 1.0 mm or more, and deviation length from cutting size is 1.5 mm or more | | | | | | | | | | | | | | | |

### <Test Example 2: Evaluation of water absorption amount and visibility due to change in open porosity (open pore area)>

In order to evaluate a relationship between open porosity (open pore area), and water absorption amount and visibility, in a super absorbent pad having a plurality of openings of the same shape, an open porosity (open pore area) was changed by changing an interval (pitch) between the openings variously, and the water absorption amount, the visibility, and suitability for perforating and cutting were evaluated.

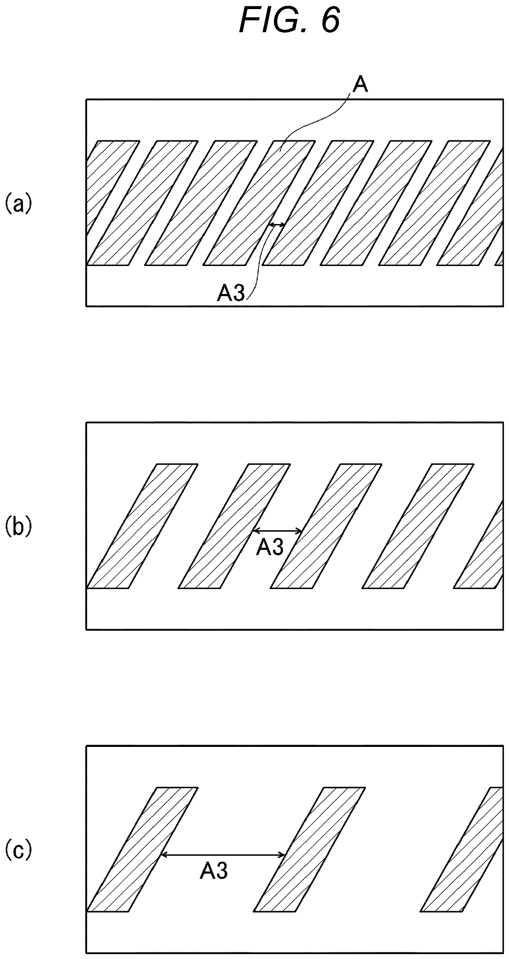

In the super absorbent pads in a wound-covering material subjected to the evaluation, by keeping the size (pad size), the pad structure, and the opening shape (size) constant as illustrated below and changing only an interval (pitch) between the openings illustrated in Table 2 (A3 in Fig. 6 (Fig. 5)), the open porosity and the open pore area of one pad were changed. Note that Fig. 6 illustrates schematic views (front views) of open pore patterns of super absorbent pads used in Example A (Fig. 6(a)), Example E (Fig. 6(b)), and Example J (Fig. 6(c)) illustrated in Table 2.
- Pad size: width in short direction (Fig. 5: 1b) 50 mm
   width in longitudinal direction (Fig. 5: 1a) 100 mm
   Pad structure: Example 1 described above (super absorbent pad A: including a surface layer and a backing layer)
- Opening shape: length (Fig. 5: A1) 10 mm x height (Fig. 5: A2) 30 mm
- Opening inclination angle (Fig. 5: θ): 40°
- Wound-covering material size (pressure-sensitive adhesive film size): width in short direction 90 mm
   width in longitudinal direction 150 mm
- Composition of pressure-sensitive adhesive film: polyurethane film (thickness) 0.018 mm
   polyurethane-based pressure-sensitive adhesive (thickness) 0.025 mm

The water absorption amount, the visibility, and the suitability for perforating and cutting were evaluated by the following procedure.

### <<Water absorption amount>>

### <Evaluation procedure>

A release material portion of a wound-covering material was peeled off, and the resulting product was placed such that the super absorbent pad faced upward. Thereafter, the super absorbent pad was caused to absorb a saline solution adjusted to 0.9% by mass (having an opening, caused to absorb the saline solution from a side of a polyethylene net as a surface layer), and the wound-covering material was allowed to stand for two hours. After the wound-covering material was allowed to stand for two hours, the mass of the wound-covering material was measured before water absorption and after being allowed to stand for two hours after water absorption, and the amount of saline solution absorbed by the super absorbent pad of the wound-covering material was calculated in terms of mass per unit area.

By a similar procedure, the water absorption amount of a market product (having an opening) per unit area was calculated (0.16 g/cm²), and this amount was set as a target value.

### <Evaluation criterion>

×: less than 0.16 g/cm²
o: 0.16 g/cm² or more

### <<Visibility>>

### <Evaluation procedure>

As a method for evaluating visibility, for the pad size: 50 mm width used for this evaluation, a range to be observed at a wound site was set to the central 30 mm width. Evaluation was made with a ratio of the opening area (visible area) with respect to the range to be observed at the wound site (30 mm width).

### <Evaluation criterion>

×: A visible area is less than 25% with respect to a range to be observed at a wound site (30 mm width)
Δ: A visible area is 25% or more and less than 27% with respect to a range to be observed at a wound site (30 mm width)
o: A visible area is 27% or more with respect to a range to be observed at a wound site (30 mm width)

### <<Suitability for perforating and cutting>>

### <Evaluation procedure>

Regarding suitability for a perforating process, a deviation length of an interval (pitch) between openings was measured.

Regarding suitability for a cutting process, when each of the pads was cut into a prescribed size, a deviation length from the size was measured.

### <Evaluation criterion>

×: A deviation length of a pitch is 1.0 mm or more, and
a deviation length from the cutting size is 1.5 mm or more
Δ: A deviation length of a pitch is 0.5 mm or more and less than 1.0 mm, and
a deviation length from the cutting size is 1.1 mm
or more and less than 1.5 mm
o: A deviation length of a pitch is less than 0.5 mm, and
a deviation length from the cutting size is less than 1.1 mm

### [Table 2]

**[Table 2]**

| Example | Pitch*¹ (Fig. 6: A3) | Open pore area*² (cm²) | Open porosity (%) | Water absorption amount | | Water absorption amount evaluation*³ | Visibility evaluation*⁴ | Suitability for perforating and cutting*⁵ |
|---|---|---|---|---|---|---|---|---|
| | | | | Average value (g/cm²) | S.D. | | | |
| A | 4 mm | 21.0 | 42.0 | 0.157 | 0.002 | × | ○ | × |
| B | 6 mm | 19.5 | 39.0 | 0.176 | 0.002 | ○ | ○ | Δ |
| C | 8 mm | 16.5 | 33.0 | 0.183 | 0.001 | ○ | ○ | ○ |
| D | 10 mm | 15.0 | 30.0 | 0.201 | 0.005 | ○ | ○ | ○ |
| E | 12 mm | 13.7 | 27.4 | 0.201 | 0.003 | ○ | ○ | ○ |
| F | 15 mm | 12.0 | 24.0 | 0.215 | 0.001 | ○ | ○ | ○ |
| G | 18 mm | 10.5 | 21.0 | 0.219 | 0.001 | ○ | ○ | ○ |
| H | 20 mm | 9.8 | 19.6 | 0.215 | 0.002 | ○ | ○ | ○ |
| I | 25 mm | 8.2 | 16.4 | 0.224 | 0.003 | ○ | Δ | ○ |
| J | 30 mm | 7.5 | 15.0 | 0.234 | 0.001 | ○ | × | ○ |
| K | No pore | 0.0 | 0.0 | 0.285 | 0.006 | ○ | × | ○ |

| | | | | | | | | |
|---|---|---|---|---|---|---|---|---|
| *1: Open pore pattern was fixed to 10 mm (width) × 30 mm (height) and inclination angle of 40° *2: Total open pore area per pad was used. *3: Water absorption amount evaluation ×: less than 0.16 g/cm² o: 0.16 g/cm² or more *4: Visibility evaluation ×: Visible area is less than 25% with respect to range to be observed at wound site Δ: Visible area is 25% or more and less than 27% with respect to range t o be observed at wound site o: Visible area is 27% or more with respect to range to be observed at w ound site *5: Suitability for perforating and cutting o: Deviation length of pitch is less than 0.5 mm, and deviation length f rom cutting size is less than 1.1 mm Δ: Deviation length of pitch is 0.5 mm or more and less than 1.0 mm, and deviation length from cutting size is 1.1 mm or more and less than 1.5 mm ×: Deviation length of pitch is 1.0 mm or more, and deviation length from cutting size is 1.5 mm or more | | | | | | | | |

### Reference Signs List

- 1: Super absorbent pad
- 2: Water-absorbing layer
- 3: Polyester nonwoven fabric
- 4: Backing layer
- 5: Surface layer
- 10: Wound-covering material
- 20: Pressure-sensitive adhesive film
- 21: Pressure-sensitive adhesive layer
- 22: Support layer
- 30(30A, 30B): Release material
- 40: Carrier film
- 50: Intake tape
- A: Opening
- B: Half cut

## Claims

1. A wound-covering material comprising
a super absorbent pad having a laminated structure of polyester nonwoven fabric layer/water-absorbing layer, wherein
the water-absorbing layer includes a super absorbent fiber, a polypropylene fiber, and a polyethylene binder, and
the super absorbent pad has an opening passing therethrough.

2. The wound-covering material according to claim 1, wherein the water-absorbing layer includes the super absorbent fiber, the polypropylene fiber, and the polyethylene binder at a mass ratio of super absorbent fiber : polypropylene fiber : polyethylene binder = more than 30% by mass and less than 50% by mass : 40% by mass or more and less than 55% by mass : more than 10% by mass and less than 30% by mass (in which the total amount of the super absorbent fiber, the polypropylene fiber, and the polyethylene binder is 100% by mass).

3. The wound-covering material according to claim 1, wherein the super absorbent pad has a basis weight of 200 to 400 g/m² and a thickness of 1.0 to 3.0 mm.

4. A wound-covering material comprising a super absorbent pad having a laminated structure of nonwoven fabric layer/water-absorbing layer, wherein
the super absorbent pad has a plurality of openings passing therethrough over an entire surface of the pad.

5. The wound-covering material according to claim 1 or 4, wherein
each of the openings has a size of 10 to 500 mm².

6. The wound-covering material according to claim 1 or 4, wherein
each of the openings has a triangular shape, a quadrangular shape, a rectangular shape, a parallelogrammical shape, a rhombic shape, a circular shape, an elliptical shape, a crescent shape, a horseshoe shape, a torus shape, or a combination thereof.

7. The wound-covering material according to claim 1 or 4, wherein
a total area of the openings is 17% or more and less than 39% with respect to 100% of a total area of an upper surface of the super absorbent pad.
